(19) Europäisches Patentamt
European Patent Office
Office européen des brevets

(11) EP 3 878 436 A1

(12) **EUROPEAN PATENT APPLICATION**

(43) Date of publication:
**15.09.2021 Bulletin 2021/37**

(21) Application number: **20161829.5**

(22) Date of filing: **09.03.2020**

(51) Int Cl.:
*A61K 9/16* (2006.01) *A61K 31/435* (2006.01)
*A61P 33/10* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Bayer Animal Health GmbH
51373 Leverkusen (DE)**
- **Heinrich-Heine-Universität Düsseldorf
40225 Düsseldorf (DE)**

(72) Inventor: **The designation of the inventor has not yet been filed**

(74) Representative: **Tier 1 Patents
C/o Bayer Intellectual Property GmbH
Creative Campus Monheim
Bldg. 4685
Alfred-Nobel-Straße 10
40789 Monheim am Rhein (DE)**

(54) **SOFT CHEWABLE FORMED BODY FOR THE ADMINISTRATION TO ANIMALS**

(57) The invention relates to a formed body for the administration to animals and to a process for the production of the formed body for the administration to animals. The formed body serves in particular for the oral administration of pharmaceutically active substances to animals. The formed body for the administration to animals comprises one or more pharmaceutically active substance, one or more flavouring agents, one or more binding agents, one or more gel-forming agents, one or more fillers, water and glycerol.

EP 3 878 436 A1

## Description

The invention relates to a formed body for the administration to animals and to a process for the production of the formed body for the administration to animals. The formed body serves in particular for the oral administration of pharmaceutically active substances to animals.

## Prior art

The acceptance of a pharmaceutical by the pet is determined mainly by its odour and taste (Thombre, A.G., 2004, Advanced Drug Delivery Reviews 56 (10), 1399-1413). Thus, for example, the use of flavourings leads to an increase in acceptance of more than 90 % for bitter medicaments (Ahmed, I. and Kasraian, K. 2002, Advanced Drug Delivery Reviews 54 (6), 871-882). Cats favour the taste of fish, whereas dogs prefer that of beef, pork and lamb (Houpt, K.A. and Smith, S.L. 1981, Canadian Veterinary Journal 22(4), 77-85). The fact that, in spite of the high proportion of flavourings and taste improvers (Rose et al. 2008, US7348027) or in some cases also animal byproducts (Cleverly et al. 2004, WO2004016252), veterinary medicinal products are still rejected is due to the obvious discriminability to the food. In general, oral presentation forms are harder than conventional animal food or differ therefrom in their form.

The palatability of a chewable medicinal product is largely determined by the mouthfeel generated (Thombre, A.G., 2004, Advanced Drug Delivery Reviews 56 (10), 1399-1413). To generate a pleasant mouthfeel, the textures of the pharmaceutical are adapted to the preferences of the patients. In the case of dogs, soft structures are preferred considerably (Rose et al. 2008, US7348027) to hard and brittle structures, for example DE69937780 (Damon et al. 2008).

What has been developed so far are chewables based on flavoured starch extrudates. The chewables are intended for mixing with the animal food employed, without the animal then being able to select (Isele 2008, AU2008201605 B2; Kalbe 2008, EP1296655 B1). By virtue of the high proportion of flavourings, the palatability is very good; however, this cannot be taken as granted for the entire storage period. Since starch-containing chewables get harder over time, as time passes, the mouthfeel generated is also perceived as more crumbly (Keetels, C.J.A.M. et al. 1996, Food Hydrocolloids 10 (3), 343-353).

A further disadvantage of starch-containing chewables is the fact that it is not possible to embed thermolabile active compounds therein. This also applies to chewables produced from a sugar mixture heated under high pressure (Han, Y.D. and Park, J.B. 2002, US6444218; Han, Y.D. and Park, J.B. 2002, US6440450).

By retrogradation of starch-containing products, previously bound water is released again, resulting in a significant change of the physical structure (Farhat et al. 2001, Starch/Stärke 53 (9), 431-436) and thus also the elasticity (Vandeputte et al. 2003. Journal of Cereal Science, 38 (19, 61-68) of the extrudates. To avoid these problems, very complicated recipes were developed which, in addition to the starch, contain a vegetable oil, sugar and further additives (Huron, S. 2004, WO2004/014143 A1; Paulsen et al. 2011, US7955632 B2). However, the oil, which is intended to improve the mouthfeel, causes problems during further processing of the chewables. For example, adhesion to tabletting tools or forming machines (Carrillo, B. and Freehauf, K. 2013, WO2013068371 A1) have been described. Moreover, during storage, the oil is separated from the remainder of the formulation, so that these chewables, too, harden and the mouthfeel gets chalky over time. Hydrophobic active pharmaceutical ingredients (API) may further accumulate in the oil phase, which can have a detrimental effect on the drug delivery process of these chewables.

In an attempt to prepare chewables of largely constant consistency and attractiveness, other starch-free chewables have been developed (Gao et al. 2010, US20100291245; Hamann, H.J. and Kanikanti, V.-R. 2012, WO2012049156 A1). However, owing to their great hardness, it is not possible to reconcile the texture of these chewables with the preferences of dogs. The texture is brittle and the mouthfeel is crumbly. In addition, the preparation of chewables according to Gao require a drying process of several hours until the chewable has reached the desired moistness.

In US 2008/0075759 A1, a process for manufacturing chewable dosage forms for drug delivery to an animal or human subjects and products thereof are described. The disclosed process avoids extrusion and elevated temperatures associated therewith, but requires separate wet mixing and forming steps for the production of chewables. WO 2014/141223 A1 relates to a chewable formulation for delivering a pharmaceutically active agent to an animal target. The chewable formulation, which may be formed by extrusion, is substantially free from unbound water and requires addition of a lipid or fat in the production process. Further palatable soft chew veterinary composition for oral administration to an animal is disclosed in WO 2016/073347 A1. The method for producing this soft chew composition, however, requires passing the extrudate through a conditioning chamber having a specific temperature range or air drying of the extrudates. In WO 2017/194415 A1, a method for the production of molded bodies for administration to animals is disclosed. The chews obtained by this method, however, lack the required mechanical stability and tend to crumble while handling the chews. Further, the water activity aw is not in accordance with the limits provided in the USP 39, 1112 (United States Pharmacopeia).

## Object of the invention

Against this background, it was an object of the present invention to provide formed bodies for the administration to animals, in particular dogs, in particular for the administration of pharmaceutically active substances, which avoid or ameliorate one or more of the above-described disadvantages of the prior art. Likewise, a further object was to provide an improved process for the production of such formed bodies. In particular, it was an object of the present invention to provide a chewable medicinal product (herein below also referred to as chewables or soft chewables) which stands out from the known products in particular by one or more of the following properties:

1) High palatability is to be achieved by the chewable medicinal product having a pleasant taste and a texture profile well-accepted by dogs, ensured by the following characteristics:

  a. substantially no breaking of the chewables at a certain force load,
  b. sufficient elasticity and plasticity at a certain force load,
  c. the dog should chew the chewable about as often as a piece of meat sausage (on average at least 3 times).

2) The chewable medicinal product should have a pleasant mouthfeel. Chewing should lead neither to a chalky nor to a crumbly feel.

3) The disintegration time of the chewable medicinal product should be below 1 hour.

4) The chewable medicinal product should have a water activity that is sufficiently low to prevent growth of microorganisms in the formed body

5) The storage time should have a negligible effect on the properties of the medicinal product, for example

  a. the disintegration time should stay below 1 hour,
  b. the release properties should not deteriorate,
  c. the pieces formed in the process of the present invention should remain elastic and firm to the bite.

6) The preparation process should meet at least most of the following requirements:

  a. robustness,
  b. preferably continuous processing,
  c. short processing time,
  d. no adhesion to process parts,
  e. curing not required,
  f. can be carried out at temperatures below 80°C.

Some or all of these objects are achieved with the formed body according to claims 1 and 13 and the process for making the formed body according to claim 14.

Preferred embodiments of the invention are described in the dependent claims and are subsequently described.

## Description of the invention

The present invention is directed towards a formed body for the administration to animals comprising:

a. one or more pharmaceutically active substances,
b. one or more flavouring agents,
c. one or more binding agents,
d. one or more gel-forming agents,
e. one or more fillers,
f. water and
g. glycerol.

Surprisingly, a formed body comprising at least the above-named combination of components possesses an extraordinary palatability with a texture that is particularly well accepted by animals, in particular by dogs and cats. The inventive formed body exhibits a meat-like, semi-solid, soft texture and can be chewed readily by animals such as dogs

and cats while providing a pleasant mouthfeel to the animals. The term "semi-solid" as used herein refers to a texture, which holds its shape unless certain levels of pressure are applied. Hardness measurements performed with a texture analyser (as further described below) also provide information on textural characteristics such as elastic behaviour, plastic deformation and brittleness or lack ofbrittleness (see e.g. Szczesniak, A. S. (1963), Classification of textural characteristics, Journal of Food Science 28(4): 385-389).

In addition to the exceptional textural properties of the inventive formed body, all its components are well distributed in the body and provide for a homogenous delivery of the contained pharmaceutically active substance in the animal. The inventive formed body furthermore exhibits a pleasant smell and can easily be handled by a pet owner.

The inventive formed body exhibits a long shelf-life even if stored over extended periods of times, e.g. for longer than two months or for longer than three months or for longer than six months. In particular, the hardness of the inventive formed body remains substantially stable during storage for several months.

Substantially stable herein means that the hardness of the formed body does not change by more than 50 %, preferably not by more than 30 %, most preferably not by more than 20 %, when comparing hardness measurements of the formed body after one week of preparation with measurements of the formed body after several months of storage, e.g. after 3 months or after 6 months. The skilled person knows how to perform such hardness measurements, which typically require a texture analyser, e.g. a TA.XTplus, Stable Micro Systems Ltd., UK. This is an important advantage of the formed body of the present invention over primarily starch-based chewable bodies of the prior art.

The disintegration time of the inventive formed body is below 1 h, preferably below 45 min and most preferably below 30 min. The disintegration time of the inventive formed body can be determined by method A of European Pharmacopeia (Ph. Eur.) 10.2. It was found that the disintegration time of the inventive formed body remains substantially equal even after storage of the formed body for a period of up to 3 months, preferably up to 6 months, more preferably up to 12 months.

With the inventive formed body, a vast array of different pharmaceutically active substances can be delivered to animals, in particular to dogs and cats, in an efficient and comfortable manner.

Without being bound to any particular scientific theory, it is believed that the interaction between the one or more gel-forming agents, water and glycerol provides the basic network structure of the inventive formed body which is partly responsible for the extraordinary properties of the formed bodies. Gel-forming agents, water and glycerol in the inventive formed body form a gel-like structure into which the other components of the body are integrated with the help of the one or more binding agents. For this reason, the inventive formed body has a homogenous, stable structure that does not change significantly even over long storage periods.

Surprisingly, it was found that components, typically used as disintegrants for tablet disintegration, have optimal gel-forming properties and thus serve as gel-forming agents in the inventive formed body. This could possibly be due to the fact that the disintegrants used as gel-forming agents in the inventive formed body swell fast in contact with water, which is believed to be the mechanism by which these disintegrants impart the disintegrating effect. This swelling capability could be a main reason for their good gel-forming properties in the inventive formed body.

The inventive formed body can be prepared with the inventive preparation process. The inventive process exhibits a high through-put rate, short residence times, and a high robustness.

For the purpose of the present invention, a formed body is to be understood as an object obtained by a formative process, preferably by cutting a strand obtained by extrusion. The formed bodies preferably have a smooth surface, with no tears visible to the naked eye and no irregularities that can be felt by hand. The weight of a formed body typically ranges from 0.1 g to 20 g, preferably from 0.5 g to 10 g and most preferably from 1 g to 5 g.

The formed bodies according to the present invention are for the administration to animals, and they can be employed in animal husbandry and animal breeding for farm animals, breeding animals, zoo animals, laboratory animals, research animals and pets, and in particular for mammals.

The farm animals and breeding animals include mammals such as, for example, cattle, horses, sheep, pigs, goats, camels, water buffalos, donkeys, rabbits, fallow deer, reindeer, fur-bearing animals, such as, for example, minks, chinchilla, racoon, and also birds such as, for example, chicken, geese, turkeys, ducks, pigeons and ostriches. Examples of preferred farm animals are cattle, sheep, pig and chicken.

Laboratory animals and research animals include dogs, cats, rabbits and rodents such as mice, rats, guinea pigs and golden hamsters.

The pets include dogs, cats, horses, rabbits, rodents such as golden hamsters, guinea pigs, mice, furthermore reptiles, amphibians and birds that are kept at home or in a zoo. Administration to cats and dogs is particularly preferred.

Since the formed bodies according to the invention are administered to animals orally and are usually chewed during administration, the terms "chewable" or "chewables", either in combination with the term "formed bodies" or alone are also used when making reference to the formed bodies according to the invention. The chewable formed bodies are further denoted as being "soft", as they have a soft consistency in comparison to prior art orally administered solid medications for animals, thus allowing to swallow the formed bodies without or with only little chewing, and provide a meat-like mouthfeel to the animals to which the chewable formed bodies are administered. Accordingly, the terms "formed

bodies", "chewable formed bodies", "chewable" or "chewables" and "soft chewable formed bodies" may all be used interchangeably in the context of this application for the object of the invention, which are soft chewable formed bodies for the administration to animals.

For the purpose of the invention, extrusion or an extrusion process is to be understood as a mixing and/or forming process utilising an extruder that is the continuous squeezing of solid to highly viscous materials under pressure from a formative opening, also referred to as die, matrix or nozzle. The resulting bodies, having a cross section which corresponds to the opening, and of theoretically any length, are accordingly referred to as extrudates.

According to the invention, mixing of the solid powder components can take place after charging of the extruders with the solid components via an inlet opening, for example via an inlet funnel. Further components of the extrusion mixture including liquids may also be introduced into the extruder via this inlet opening or other inlet openings at different locations, either before, during or after charging the extruder with the solid components via an inlet opening. Homogenization and plastification during transport through the frequently stepped screw in the extruder may take place with heating or cooling, until finally the material is squeezed through the formative die in the head of the extruder.

For the purpose of the invention, the term extruder comprises mono-, twin- and multi-screw extruders, planetary roller extruders and cascade extruders, without being limited to these extruder variants.

The inventive formed body comprises water which is important for the texture and disintegration properties of the formed body. According to a preferred embodiment of the inventive formed body, the water content of the formed body is in the range of 0.5 to 20 wt.%, preferably 0.75 to 18 wt.%, more preferably 1.0 to 15 wt.%, even more preferably 1.5 to 13 wt.%, and most preferably 1.75 to 11.5 wt.%, based on the total weight of the formed body. It has been found that a formed body with such preferred water content shows an extended shelf-life and provides a particularly stable but soft texture for the formed body. In addition, formed bodies with the aforementioned preferred water content exhibit excellent workability and can be easily processed through extrusion processes. The value of water activity aw of the formed body is preferably below or equal to 0.6, further preferred in the range of from 0.10 to 0.6, particularly preferred in the range of from 0.20 to 0.58, and most preferred in the range of from 0.30 to 0.55. In case the water activity of the formed body is within such a preferred range, the formed body exhibits a particularly long storability. The inventors have found that the growth of microorganisms in the formed body is inhibited at such low levels of water activity.

The ranges indicated herein for each component of the inventive formed body can be combined with any range indicated for another component of the formed body. In particular, the ranges of the same "preference level" are compatible for different components of the formed body.

The water activity (aw) is the partial vapor pressure of water in the formed bodies divided by the partial vapor pressure of pure water at the same temperature. This relationship is displayed by the following equation

$$aw = p/p^*$$

wherein p is the vapor pressure of water in the formed body, and p* is the vapor pressure of pure water at the same temperature.

The values of aw of the formed body given for this preferred embodiment of the invention refer to measurement of aw at 22 °C. For measurement of aw, resistive electrolytic hygrometers, capacitance hygrometers or dew point hygrometers can be used.

The water activity of the formed bodies depends on the water content, but also on the kind of gel-forming agents, further liquids and excipients used in the production of the soft chewable formed bodies. By keeping the value for water activity aw at a range as indicated herein, microbial growth is prevented, and storability of the formed bodies is enhanced.

The inventive formed body furthermore comprises one or more binding agents. Preferably one binding agent is used. The one or more binding agents can be selected from the group of polyvinylpyrrolidone, croscarmellose, hydroxypropylcellulose, hydroxypropylmethyl cellulose, hydroxyethylcellulose, pectin, pullulan, carrageenan, xanthan gum, alginate and agar agar. It has been found that the texture and the disintegration properties of the formed body are particularly favourable if the one or more binding agents comprise or consist of polyvinylpyrrolidone. With polyvinylpyrrolidone as binding agent, the one or more pharmaceutically active substances, the one or more flavouring agents and possible additives are particularly well distributed in the gel-network of the formed body.

Polyvinylpyrrolidones (PVP, povidones) are commercially available hydrophilic polymers suitable for use in solid pharmaceutical preparations. Various types of PVP are commercially available. PVP of relatively low molecular weight are normally employed as binders for tablets. PVP swell in aqueous medium and erode. It is possible by using PVP having different molecular weights to vary the kinetics of release within a defined range in the inventive bodies.

The polyvinylpyrrolidones or polyvinylpyrrolidone derivatives employed are preferably soluble in water. In this case, the polyvinylpyrrolidones or polyvinylpyrrolidone derivatives are preferably substantially linear and not crosslinked.

In general, any type of polyvinylpyrrolidone can be used as binding agent for the formed body. According to a preferred embodiment, the formed body comprises a polyvinylpyrrolidone that has a weight average molecular weight

$M_w$ of from 1,000 to 500,000 g/mol, in particular from 1,200 to 300,000 g/mol, from 1,500 to 100,000 g/mol or from 2,000 to 90,000 g/mol. Polyvinylpyrrolidone with such a weight average molecular weight has been found to be particularly well suited for integrating the different components of the formed body into the gel-like structure. Polyvinylpyrrolidone that has a weight average molecular weight of more than 500,000 g/mol can result in formed bodies that have a more brittle, less soft general structure due to curing effects that can take place with such very high molecular weight polyvinylpyrrolidone. In contrast, polyvinylpyrrolidone with a weight average molecular weight of less than 1,000 g/mol exhibits a less than ideal binding effect of components into the gel-structure.

According to a preferred embodiment of the invention, the formed body comprises polyvinylpyrrolidone having a K value of 17 to 90, preferably from 17 to 60, most preferred from 25 to 50.

The K value of the polyvinylpyrrolidones or polyvinylpyrrolidone derivatives is related to the viscosity and the molecular weight and can be determined by methods known to the skilled person. If in doubt, the data on the K value from the Ph. Eur. are used. One possible way of calculating the K value is by using the K value by Fikentscher. At constant measurement conditions regarding solvent, concentration of polymer in solution and temperature, the K value according to Fikentscher is only depending on the molecular mass of the polymer. The K value by Fikentscher is calculated by

$$K = 1000 \times k = 1000 \times \frac{1{,}5 lg\eta_r - 1 + \sqrt{1 + \left(\frac{2}{c} + 2 + 1{,}5 lg\eta_r\right) \times 1{,}5 lg\eta_r}}{150 + 300c}$$

wherein $\eta_r$ is the relative viscosity (which equals the dynamic viscosity of the solution divided by the dynamic viscosity of the solvent) of the solution and c is the concentration by mass in g/cm$^3$ of the polymer in solution. Details regarding the calculation and measurement of the K value with a capillary viscometer can be found in norm DIN EN ISO 1628-1.

According to one embodiment of the invention, the formed body only comprises one type of polyvinylpyrrolidone. Such a formed body can be particularly cheap to produce while exhibiting good textural and disintegration properties.

According to another embodiment of the invention, the one or more binding agents comprise or consist of at least two different types of polyvinylpyrrolidone. According to a particularly preferred embodiment, the one or more binding agents comprise or consist of at least two types of polyvinylpyrrolidone having different weight average molecular weights. In case the formed body comprises two or more different types of polyvinylpyrrolidone having different weight average molecular weights as binding agents, the different types of polyvinylpyrrolidone can provide a formed body with fined-tuned properties according to the used specific ingredients and the specific intended use.

A type of polyvinylpyrrolidone is to be understood herein as a polyvinylpyrrolidone with a particular weight average molecular mass, particular rheological properties such as viscosity and/or particular mechanical properties that are different from at least one other type of polyvinylpyrrolidone. Hence, different types of polyvinylpyrrolidone within the meaning of the invention may differ with regards to their weight average molecular mass, their rheological properties such as viscosity and/or their mechanical properties. For example, one type of polyvinylpyrrolidone might be defined by having a weight average molecular mass of 2,000 to 6,000 g/mol, whereas another different type of polyvinylpyrrolidone might have a weight average molecular mass of 20,000 to 50,000 g/mol.

According to a preferred embodiment of the invention, the content by weight of polyvinylpyrrolidone in the formed body is in the range of from 0.2 to 20.0 wt.%, preferably from 0.3 to 10.0 wt.%, more preferred from 0.4 to 9.0 wt.%, and most preferred from 0.5 to 7.5 wt.%, based on the total weight of the formed body. With such a weight content of polyvinylpyrrolidone, the inventive formed body shows particularly good textural properties while all components are well distributed in the body. With a weight content of more than 20 wt.% polyvinylpyrrolidone, there is a risk that the formed body loses its desired properties. In case the weight content of polyvinylpyrrolidone is less than 0.2 wt.%, it may be difficult to obtain a completely homogenous formed body into which all components are well distributed.

Details concerning the abovementioned polyvinylpyrrolidones, polyvinylpyrrolidone derivatives and particular mixtures can be found in the following book: V. Bühler, "Kollidon, Polyvinylpyrrolidone for the pharmaceutical industry", 9th revised edition, BASF Pharma Ingredients, Germany, 2008.

The inventive formed body comprises one or more gel-forming agents. The gel-forming agents used in the inventive formed body are typically used as disintegrants in solid tablets. In general, different types of gel-forming agents can be used for the formed body, preferably one or more gel-forming agents selected from the group of croscarmellose, low substituted carboxymethylcellulose, low substituted hydroxypropylcellulose, crospovidone and cross-linked carboxymethylstarch. Experimental studies have shown that a particularly robust and soft formed body can be achieved when the one or more gel-forming agents comprise or consist of croscarmellose. Croscarmellose as referred to herein is also known as cross-linked carboxymethylcellulose. It was found that croscarmellose is exceptionally well suited for providing a soft but robust network structure in combination with water and glycerol, into which the different flavouring agents and active pharmaceutical substances as well as optional additives can be integrated. A formed body in which

the one or more gel-forming agents comprise or consist of croscarmellose has a particularly long shelf-life and exhibits good disintegration properties.

It is particularly preferred that croscarmellose is the only gel-forming agent in the formed body. In this manner, a particularly homogenous formed body can be obtained.

In a preferred embodiment according to the invention, no gelatine is contained in the formed body.

In principal, the gel-forming agents used for the inventive formed body can also be used as binding agents. Preferably, the one or more binding agents and the one or more gel-forming agents differ from each other by at least one agent. Particularly preferred is to use the combination of croscarmellose as gel-forming agent and polyvinylpyrrolidone as binding agent in the inventive formed body. Thus, in this embodiment of the inventive formed body, the gel-forming agents comprise or consist of croscarmellose and the binding agents comprise or consist of polyvinylpyrrolidone. Another preferred combination is to use crospovidone as gel-forming agent with hydroxypropylcellulose as binding agent in the inventive formed body. Thus, in this embodiment of the inventive formed body, the gel-forming agents comprise or consist of crospovidone and the binding agents comprise or consist of hydroxypropylcellulose.

In another preferred embodiment of the invention, substantially no starch and/or starch derivative is contained in the formed body. In another preferred embodiment, the inventive formed body is free of starch and/or starch derivative. By having substantially no starch and/or starch derivative in the formed body, it is ensured that the hardness of the formed body remains particularly stable over long storage periods.

Croscarmellose is a salt, preferably a sodium salt, of a cross-linked, partly O-(carboxymethylated) cellulose. Croscarmellose is well known to the skilled person. The degree of substitution of the cellulose structure, i.e. how many of the hydroxyl groups per glucopyranose monomer unit are replaced with carboxymethyl groups, can vary broadly. A typical degree of substitution may preferably be from 0.5 to 0.9 carboxymethyl groups per glucopyranose unit, in particular around 0.7 carboxymethyl groups per glucopyranose unit. Croscarmellose can be prepared by first soaking crude cellulose in sodium hydroxide, then reacting the cellulose with sodium monochloroacetate to form sodium carboxymethylcellulose. Excess sodium monochloroacetate then slowly hydrolyzes to glycolic acid which catalyzes the crosslinkage to form sodium croscarmellose. Other techniques for crosslinking are possible.

It is believed that the pleasant mouthfeel of the inventive formed body can be attributed for a significant part to the gel-like structure provided by the network of one or more gel-forming agents with glycerol and water integrated. The quality of the gel-like structure depends *inter alia* on the ratio of these components to each other. The formed body possesses a particularly stable and robust network structure when the weight ratio of the one or more gel-forming agents to the combined weight of water and glycerol in the formed body is in the range of from 1:2 to 1:20, preferably from 1:3 to 1:15, more preferred from 1:5 to 1:10 and most preferred from 1:6 to 1:8. It is believed that with such a weight ratio of one or more gel-forming agents to the combined weight of water and glycerol, the gel structure has the optimal ratio of solid and liquid components to provide for a particularly robust network structure. In case the weight ratio of one or more gel-forming agents to the combined weight of water and glycerol in the formed body is above 1:2, the texture of the formed body can become brittle and less soft. With a weight ratio of one or more gel-forming agents to the combined weight of water and glycerol of less than 1:20, there is a risk that not all components of the formed body are equally well distributed in the formed body.

According to another preferred embodiment of the invention, the content by weight of the one or more gel-forming agents in the formed body is in the range of from 0.5 to 30 wt.%, preferably from 1 to 25 wt.%, more preferred from 2 to 20 wt.%, even more preferred from 3 to 17 wt.% and most preferred from 5 to 15 wt.%, based on the total weight of the formed body. With such a content of one or more gel-forming agents, the formed body exhibits particularly favourable textural properties and a particular long shelf-life.

According to a particularly preferred embodiment, the weight ratio of one or more gel-forming agents to the combined weight of water and glycerol in the formed body is in the range of from 1:2 to 1:20, preferably from 1:3 to 1:15, more preferred from 1:5 to 1:10 and most preferred from 1:6 to 1:8 and simultaneously, the content by weight of the one or more gel-forming agents in the formed body is in the range of from 0.5 to 30 wt.%, preferably from 1 to 25 wt.%, more preferred from 2 to 20 wt.%, even more preferred from 3 to 17 wt.% and most preferred from 5 to 15 wt.%, based on the total weight of the formed body.

The inventive formed body comprises glycerol and water. Surprisingly, when only glycerol is used, the one or more gel-forming agents are not able to form a suitable network structure, in particular in case croscarmellose is used as gel-forming agent. However, when a mixture of glycerol and water is used together with one or more gel-forming agents, in particular croscarmellose, for the formed body, a soft and robust gel-like structure is obtained. The ratio of glycerol and water in the inventive formed body can vary in broad ranges. With a formed body having a weight ratio of water to glycerol in the formed body from 5:95 to 35:65, preferably from 10:90 to 30:70 or most preferred from 15:85 to 25:75, the structural integrity of the gel-like structure is particularly high. In case the weight ratio of water to glycerol is below 5:95, the structural integrity of the gel-like structure of the formed body can suffer. If, however, the weight ratio of water to glycerol is more than 35:65, it can have a negative effect on the chewability of the formed body.

The inventive formed body has a soft but firm structure. The formed body is particularly well suited for the

administration to animals, when the formed body possesses a hardness of from 1.5 to 10N, in particular from 2.5 to 7 N. According to this invention, the hardness is defined as the maximum force at 25 % deformation ("strain") of the sample. Ways to measure the hardness of a formed body are known to the skilled person. For example, the hardness may be measured with a TA.TXplus systems from Stable Micro Systems Ltd., UK. In this case, a formed body is compressed under defined conditions and the forces necessary to achieve a certain deformation of the formed body are calculated with a suitable software such as the "Exponent"-Software.

The inventive formed body comprises one or more pharmaceutically active substances. Preferably, the at least one of the one or more pharmaceutically active substances a) is selected from the group of orally administrable systemically active veterinary pharmaceuticals, such as antiparasitic agents, in particular insecticides, acaricides, anthelmintics, and antimicrobial agents. Thus, according to the invention, pharmaceutically active substances which may be contained in the formed bodies according to the invention are, in principle, all possible active compounds which are commonly administered orally to animals.

The active compounds comprise, for example, antimicrobially active compounds, such as antiviral active compounds, antibiotic active compounds and those acting against protozoa, such as coccidia; furthermore, for example, anti-inflammatory and psychotropic active compounds and also proton pump inhibitors etc., and also particularly preferably active compounds acting against parasites (ectoparasites and/or endoparasites), such as acaricidal, insecticidal, anthelmintic active compounds.

In a preferred embodiment, the pharmaceutically active substance(s) a) are antimicrobial substances or antibiotics, preferably for the treatment of bacterial disorders.

In another preferred embodiment the pharmaceutically active substance(s) a) are antiparasitics.

In another preferred embodiment, the pharmaceutically active substance(s) a) are active compounds against coccidia.

In a particularly preferred embodiment, the antiparasitics are ectoparasiticides, in particular arthropodicides, namely insecticides or acaricides, or endoparasiticides, in particular anthelminthics.

In a very particularly preferred embodiment, the antiparasitics are insecticides, acaricides or anthelminthic s.

Examples of suitable active compounds are the following known classes: acaricides, such as the macrocycles abamectin, doramectin, eprinomectin, ivermectin, milbemectin, nikkomycins, selamectin, tetranactin and thuringiensin; bridged diphenyl acaricides such as azobenzene, benzoximate, benzyl benzoate, bromopropylate, chlorbenside, chlorfenethol, chlorfenson, chlorfensulphide, chlorobenzilate, chloropropylate, dicofol, diphenyl sulfone, dofenapyn, fenson, fentrifanil, fluorbenside, proclonol, tetradifon and tetrasul; carbamate acaricides such as benomyl, carbanolate, carbaryl, carbofuran, fenothiocarb, methiocarb, metolcarb, promacyl and propoxur; oximecarbamate acaricides such as aldicarb, butocarboxim, oxamyl, thiocarboxim and thiofanox; dinitrophenol acaricides such as binapacryl, dinex, dinobuton, dinocap, dinocap-4, dinocap-6, dinocton, dinopenton, dinosulfon, dinoterbon and DNOC; formamidine acaricides such as amitraz, chlordimeform, chloromebuform, formetanate and formparanate; growth regulators for mites such as clofentezine, dofenapyn, fluazuron, flubenzimine, flucycloxuron, flufenoxuron and hexythiazox; organochlorine acaricides such as bromocyclen, camphechlor, dienochlor and endosulfan; pyrazole acaricides such as acetoprole, fipronil and analogues and derivatives thereof, tebufenpyrad, pyriprole and vaniliprole; pyrethroid acaricides such as, for example, pyrethroid ester acaricides such as acrinathrin, bifenthrin, cyhalothrin, cypermethrin, alpha-cypermethrin, fenpropathrin, fenvalerate, flucythrinate, flumethrin, fluvalinate, tau-fluvalinate and permethrin, pyrethroid ether acaricides such as halfenprox; quinoxaline acaricides such as quinomethionate and thioquinox; sulfite ester acaricides such as propargite; tetronic acid acaricides such as spirodiclofen; and acaricides not belonging to a particular class, such as acequinocyl, amidoflumet, arsenic oxide, chlormethiuron, closantel, crotamiton, diafenthiuron, dichlofluanid, disulfiram, fenazaflor, fenazaquin, fenpyroximate, fluacrypyrim, fluenetil, mesulfen, mnaf, nifluridide, pyridaben, pyrimidifen, sulfiram, sulfluramid, sulfur and triarathene.

Insecticides may belong to various chemical classes, such as, for example, chlorinated hydrocarbons, organophosphates, carbamates, pyrethroids, formamidines, borates, phenylpyrazoles and macrocyclic lactones. Known insecticides include imidacloprid, fenthion, fipronil, allethrin, resmethrin, fenvalerate, permethrin, malathion and derivatives thereof. According to one embodiment, insecticides of the neonicotinoid class are preferred, for example acetamiprid, clothianidin, dinotefuran, imidacloprid (see above), nitenpyram, thiacloprid and thiamethoxam. Frequently used growth-regulating active compounds (insect growth regulators, IGRs) are, for example, benzoylphenyl ureas, such as diflubenzuron, lufenuron, noviflumuron, hexaflumuron, triflumuron and teflubenzuron or active ingredients such as fenoxycarb, pyriproxyfen, methoprene, kinoprene, hydroprene, cyromazine, buprofezin, pymetrozine and derivatives thereof.

Anthelmintics may be endoparasiticides or endectocides and encompass the following well-known groups: macrocyclic lactones, benzimidazoles, probenzimidazoles, imidazothiazoles, tetrahydropyrimidines, organophosphates, piperazines, salicylanilides and cyclic depsipeptides (see below).

Preferred anthelmintics encompass macrocyclic lactones having a broad spectrum, such as avermectins, milbemycins and derivatives thereof, such as, for example, ivermectin, doramectin, moxidectin, selamectin, emamectin, eprinomectin, milbemectin, abamectin, milbemycin oxime, nemadectin and derivatives thereof. The classes of the benzimidazoles, benzimidazole carbamates and probenzimidazoles also encompass active compounds such as thiabendazole, mebendazole, fenbendazole, oxfendazole, oxibendazole, albendazole, luxabendazole, netobimin, parbendazole, flubendazole, cyclobendazole, febantel, thiophanate and derivatives thereof. Imidazothiazoles encompass active compounds such as tetramisole, levamisole and derivatives thereof. The tetrahydropyrimidines encompass active compounds such as morantel, pyrantel and derivatives thereof. Organophosphates encompass active compounds such as dichlorvos, haloxon, trichlorfon and derivatives thereof. Salicylanilides encompass active compounds such as closantel, tribromsalan, dibromsalan, oxyclozanide, clioxanide, rafoxanide, brotianide, bromoxanide and derivatives thereof. Cyclic depsipeptides encompass compounds having 6 to 30 ring atoms and are composed of amino acids and hydroxycarboxylic acids as structural units of the ring.

Antimicrobial compounds are, for example, various penicillins, tetracyclines, sulfonamides, cephalosporins, cephamycin, aminoglucosides, trimethoprim, dimetridazole, erythromycin, framycetin, fruazolidone, various pleuromutilins such as tiamulin, valnemulin, various macrolides, streptomycin, clopidol, salinomycin, monensin, halofuginone, narasin, robenidine, quinolones, etc. Specific examples of fluoroquinolones include benofloxacin, binfloxacin, cinoxacin, ciprofloxacin, danofloxacin, difloxacin, enoxacin, enrofloxacin, fleroxacin, ibafloxacin, levofloxacin, lomefloxacin, marbofloxacin, moxifloxacin, norfloxacin, ofloxacin, orbifloxacin, perfloxacin, temafloxacin, tosufloxacin, sarafloxacin and sparfloxacin. A further example of an antibacterial fluoroquinolone that can be used in animals is pradofloxacin. Specific examples of other quinolones include pipemidic acid and nalidixic acid.

Apart from the above-mentioned active pharmaceutical compounds, it is also possible to have vitamins or minerals, for example, as constituents.

The active ingredients may preferably be, for example, depsipeptides selected from the group consisting of PF 1022A and emodepside.

Preferred antimicrobial fluoroquinolones are in particular enrofloxacin or pradofloxacin.

In a further preferred embodiment, the formed bodies according to the invention contain an active ingredient selected from febantel, pyrantel (typically in the form of a salt, the embonate being preferred) and praziquantel or a two-part combination composed of said active ingredients. Even more preferably, febantel, pyrantel embonate and praziquantel are used as a three-part combination in the formed bodies according to the invention.

The active compounds can also - where applicable - be used in the form of their salts with pharmaceutically acceptable acids or bases or else as solvates, more particularly hydrates, of the active compounds or their salts.

Prodrugs of the active compounds can also be used.

In a preferred embodiment, the solid active compounds used as ingredients a) are present at a mean particle size D(50) of 100 nm to 50 μm, preferably 100 nm to 10 μm. For the purpose of the present invention, D(50) is understood as a volume-based particle size distribution at which 50% of all particles have a dimension (diameter) smaller than or equal to this value. The particle sizes indicated here are determined by the laser diffraction method using the Mastersizer 2000 apparatus (Hydro 2000G dispersion unit) from Malvern and the Fraunhofer diffraction evaluation mode, since the refractory indices of the active compound particles are unknown. Here, with stirring, a suitable amount of the sample is predispersed with 2-3ml of a dispersing medium (low-viscosity paraffin). The dispersion is then introduced into the dispersion unit of the apparatus and measured. Depending on the setting, the evaluation software states the particle size as D(50) values, D(10) values, D(90) values, etc.

According to the invention the formed bodies contain at least one active compound in a pharmaceutically effective amount, where "pharmaceutically effective amount" represents a non-toxic amount of active compound which can cause the desired effect. The amount of active compound employed depends on the active compound, the treated animal and the nature, the severity and the stage of the disease.

In a preferred embodiment according to the invention, the inventive formed body contains one or more flavouring agents, preferably meat flavouring agents. Meat flavouring agent refers to an additive which is of synthetic or animal origin or a mixture of the two and which confers a meat-like odour and/or taste to the formed bodies according to the invention. Particularly preferably, use is made of meat flavouring agents purely of animal origin. These are prepared, for example, from beef, poultry, fish, animal skins or animal livers. Even more preference is given to desiccated liver powder, for example from cattle, sheep, poultry or pig and very particularly preferably from poultry or pig.

The inventive formed body can comprise fillers d) which can reduce the costs and further improve the textural properties of the formed body. According to a preferred embodiment of the invention, the formed body comprises one or more fillers selected from lactose, cellulose, and poorly soluble inorganic salts. According to the invention, poorly soluble salts are salts with a solubility in 100 g of water at 20°C of less than 1 g. Dicalcium phosphate is particularly preferred as poorly soluble inorganic salt, wherein this expression in the sense of the present invention comprises both dicalcium phosphate anhydrous and dicalcium phosphate dihydrate.

In another preferred embodiment according to the invention, one or more fillers d) comprised by the formed body are selected from solid sugar alcohols and inorganic calcium, magnesium, sodium or potassium salts.

In a particularly preferred embodiment of the process, the sugar alcohol used as filler d) is selected from the group consisting of compounds of the formula:

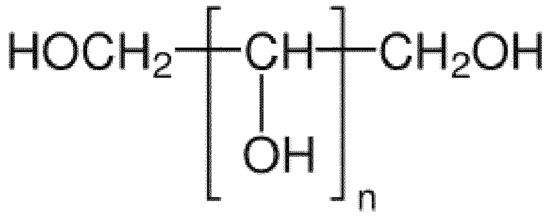

in which n ≥3 and ≤5, preferably n = 3 or 4.

In a very particularly preferred embodiment of the process, the sugar alcohol used as filler d) is mannitol, xylitol or sorbitol.

According to a preferred embodiment, the inventive formed body comprises one or more fillers selected from the group of lactose, cellulose, poorly soluble inorganic salts, in particular dicalcium phosphate, solid sugar alcohols, in particular mannitol, xylitol or sorbitol and inorganic calcium, magnesium, sodium or potassium salts.

In a preferred embodiment of the invention, the formed body comprises one or more surfactants. Preferred surfactants are non-ionic amphiphilic agents. A formed body according to the invention comprising one or more surfactants, preferably non-ionic amphiphilic agents, has been found to exhibit a particularly good bioavailability of the pharmaceutically active substances.

According to the invention, non-ionic amphiphilic agents are chemical compounds possessing both hydrophilic, i.e. water-loving or polar, and lipophilic, i.e. fat-loving or non-polar, properties, wherein the non-ionic amphiphilic agents, in contrast to cationic, anionic or zwitter-ionic amphiphiles, do not contain charged groups. Typically, non-ionic amphiphilic agents are characterized by their uncharged, hydrophilic headgroups. Examples for non-ionic amphiphilic agents according to the invention, without being limited thereto, are detergents which comprise polyoxyethylene chains as the hydrophilic part. For example, esters of fatty acids and/or glycerol comprising polyoxyethylene chains may be mentioned, e.g. stearoyl polyoxyl-32 glycerides (Gelucire® 50/13 from Gattefossé) or polyethylene glycol monostearate (Gelucire® 48/16 from Gattefossé).

In a preferred embodiment according to the invention, the content of one or more surfactants, in particular non-ionic surfactants, in the soft chewable formed body is in the range of 0.1 to 15 wt.%, preferably 0.5 to 10 wt.%, more preferably 1 to 5 wt.%, and most preferably in the range of 2 to 4 wt.%. Herein, the content in wt.% of the surfactant is defined as the quotient of the mass of all surfactants contained in a formed body and the total mass of a formed body multiplied by 100. A formed body with such content of surfactant exhibits a particularly good bioavailability of the one or more pharmaceutically active substances whilst retaining a particularly pleasant mouthfeel for the animal. With a content of more than 15 wt.% of one or more surfactants in the formed body, it can become difficult to fulfil the requirements regarding the optimal hardness and textural integrity of the body. In a preferred embodiment according to the invention, the one or more surfactant, preferably non-ionic amphiphilic agent, has a hydrophilic lipophilic balance (HLB) in the range of 6 to 20, in particular in the range of 8 to 18 or in the range of 9 to 11. Such surfactants, have been found to be particularly well suited to provide an improved bioavailability while the formed body retains excellent textural properties.

In the sense of the present invention, the HLB of a surfactant, which is a measure of the degree of how hydrophilic or lipophilic a surfactant is, is determined by calculation according to *Griffin's* method. The HLB is calculated according to the equation

$$HLB = (20 * M_h) / M$$

wherein $M_h$ is the molecular mass of the hydrophilic portion of the molecule, and M is the molecular mass of the whole molecule, giving a result on a scale of 0 to 20. An HLB value of 0 corresponds to a completely lipophilic/hydrophobic molecule, and a value of 20 corresponds to a completely hydrophilic/lipophobic molecule.

The inventive formed body can comprise the different components in varying quantities. According to a preferred embodiment, the formed body contains

0.1 to 25 wt. % of one or more pharmaceutically active substances a),
1 to 25 wt.% of one or more flavouring agents b),
1 to 20 wt.% of one or more binding agents c),
0.5 to 30 wt.% of one or more gel-forming agents d),
0 to 50 wt.% of one or more fillers e)
2 to 30 wt.% water f),
5 to 65 wt.% glycerol g),
0 to 20 wt.% of one or more auxiliary additives, as for instance formulation adjuvants, lubricants, disintegrants,

surfactants, humectants and preservatives,

wherein the percentages add up to the sum of 100 wt.%. In case the formed body comprises the aforementioned components in these quantities, the formed body has a particularly pleasant mouthfeel and a long shelf-life.

According to another embodiment, the formed body consists substantially of 0.1 to 25 wt.% of one or more pharmaceutically active substances a), 1 to 25 wt.% of one or more flavouring agents b), 1 to 20 wt.% of one or more binding agents c), 0.5 to 30 wt.% of one or more gel-forming agents d), 0 to 50 wt.% of one or more fillers e), 2 to 30 wt.% water f) and 5 to 65 wt.% glycerol g), wherein "consists substantially" means that the formed body consists to at least 80 wt.%, preferably at least 85 wt.%, more preferred at least 90 wt.% or at least 97 wt.% or at least 98 wt.% or at least 99 wt.% of the aforementioned components. The remaining 20 wt.% (or 15 wt.%, or 10 wt.%, or 3 wt.% or 2 wt.% or 1 wt.%) may be auxiliary additives such as formulation adjuvants, lubricants, disintegrants, surfactants, humectants and preservatives. Though the gel-forming agent used in the present formed bodies may also have disintegrant properties (as explained above) the composition may optionally contain a further disintegrant, depending on the specific desired properties. If a further disintegrant is present as an auxiliary additive, it is typically present in an amount of from 5 to 15 wt.%, preferably 8 to 12 wt.%, and most preferably 5 to 10 wt.%.

According to a further embodiment the formed bodies of the present invention do not contain a further disintegrant as auxiliary additive. In this case the auxiliary additives are present in amounts of from 0 to 10 wt.%, or 0 to 3 wt.%, or 0 to 2 wt.%, or 0 to 1 wt.%, or 1 to 10 wt.%, or 1 to 3 wt.%, or 1 to 2 wt.%. The components a) to g) then constitute at least 90 wt.%, or at least 97 wt.%, or at least 98 wt.% or at least 99 wt.% of the formed bodies.

According to another embodiment, the formed body consists of 0.1 to 25 wt.% of one or more pharmaceutically active substances a), 1 to 25 wt.% of one or more flavouring agents b), 1 to 20 wt.% of one or more binding agents c), 0.5 to 30 wt.% of one or more gel-forming agents d), 2 to 30 wt.% water f) and 5 to 65 wt.% glycerol g).

The invention is further directed toward the inventive formed body for use in the control of parasites in non-human animals, in particular dogs and/or cats.

In the field of animal health, i.e. the field of veterinary medicine, the soft chewable formed body according to the invention may be for use against animal parasites, in particular ectoparasites or endoparasites. The term endoparasites comprises in particular helminths and protozoa such as coccidia. Ectoparasites are typically and preferably arthropods, in particular insects and acarids.

In the field of veterinary medicine, the soft chewable formed body according to the invention is suitable for controlling parasites encountered in animal breeding and animal husbandry in farm animals, breeding animals, zoo animals, laboratory animals, research animals and pets.

By employing the active compounds according to the invention for controlling animal parasites, morbidity, mortality and reduced performance (for meat, milk, wool, hides, eggs, honey and the like) are to be reduced and/or prevented, so that more economical and simpler animal husbandry is possible and wellbeing of the animals can be improved.

With respect to the field of animal health, the term "control" or "controlling" means that the active compounds can effectively control the occurrence of the parasite in question in an animal infected by such a parasite to an extent which is harmless. More accurately, in the present context "controlling" means that the active compound can kill the parasite in question, prevent its growth or prevent its multiplication.

All definitions and embodiments described with regards to the inventive formed body also apply to the formed body for use in the control of parasites in non-human animals.

The invention is furthermore directed towards a process for making the inventive formed body.

The inventive process comprises the following steps:

a) providing at least the following components:

a. one or more pharmaceutically active substances,
b. one or more flavouring agents,
c. one or more binding agents,
d. one or more gel-forming agents,
e. one or more fillers,
f. optionally one or more auxiliary additives, as for instance formulation adjuvants, lubricants, surfactants, humectants and preservatives,

b) preparing a swollen mixture by mixing the components of step a) with glycerol and water,

c) forming a formed body by extrusion of the swollen mixture prepared in step b).

With the inventive process, the formed body can be obtained efficiently with high throughput rates in a robust fashion. The inventive process can be carried out continuously, allowing for a rapid and low -cost production of the inventive formed body.

The components of step a) are provided as solids and/or liquids. In a preferred embodiment, the components of step a) are provided as solids, preferably as powders. In the case that at least one of the components of step a) is provided as liquid, said liquid typically comprises apart from the component of step a) water.

In step b) of the inventive process a swollen mixture is prepared. The term "swollen mixture" as used herein refers to a mixture of components a), water and glycerol that has been in contact for a certain time (the "swelling time", which is the overall time from the first contact of the components a) with water and/or glycerol until the forming of the body), which is preferably at least 10 seconds, more preferably at least 30 seconds, even more preferably at least 60 seconds. According to one embodiment the swelling time is 0.5 to 10 minutes, preferably 1 to 6 minutes.

In one embodiment, the swollen mixture is prepared by providing a pre-mixture comprising the components of step a) and mixing said pre-mixture with water and glycerol or with a pre-mixture of water and glycerol.

It is also possible to prepare the swollen mixture of step b) by providing a pre-swollen mixture comprising water, glycerol and at least one, but not all of the components of step a) and mixing to said pre-swollen mixture the remaining components of step a). The remaining components of step a) that are admixed to the pre-swollen mixture can be in the form of a solid, preferably a powder, or in the form of a liquid, which optionally comprises additional water and/or glycerol.

Depending on which solid pharmaceutically active substance is used to form the pre-swollen or swollen mixture, it is possible that the pharmaceutically active substance dissolves at least partially in the mixture of water and glycerol contained therein. Experimental studies have shown that the pharmaceutically active substance praziquantel has a high solubility in a mixture of water and glycerol. Without being bound to any particular scientific theory, it is believed that the dissolution of a pharmaceutically active substance increases its bioavailability.

The pre-mixture comprising the components of step a), the pre-swollen mixture comprising water, glycerol and at least one, but not all of the components of step a) and/or the swollen mixture of step b) is/are preferably obtained by mixing with a mixer, a kneader or an extruder. Most preferably, an extruder is used to obtain the pre-mixture comprising the components of step a), the pre-swollen mixture comprising water, glycerol and at least one, but not all of the components of step a) and/or the swollen mixture of step b).

The feed-rate of solids, preferably powders, to prepare the swollen-mixture of step b) is typically performed with a powder feeder. The feed-rate of liquids to prepare the swollen-mixture of step b) is typically performed with pumps, preferably dosing pumps.

The inventive formed bodies are formed by extrusion of the swollen-mixture obtained in step b). The extruded swollen-mixture exiting the nozzle of the extruder is typically cut into pieces.

Preferably, the process step(s) b) and/or c) is/are carried out in an extruder, most preferably in one extruder. In a preferred embodiment, the entire inventive process is an extrusion process. In this manner, the process can be carried out continuously, allowing for particularly high throughput rates and process efficiency.

According to a preferred embodiment, the temperature during steps b) and/or c) is in the range of from 10 °C to 80 °C, preferably from 15 °C to 70 °C or most preferred from 20 °C to 60 °C. By carrying out steps b) and/or c) at these temperatures, a particularly homogenous mixture can be obtained.

All definitions and embodiments described with regards to the inventive formed body also apply to the inventive process of making the formed body.

The invention will be further exemplified by the following examples which are, however, not intended to limit the scope of the invention.

## Examples

All components for the preparation of the inventive formed body were purchased from commercial sources in adequate purity and used without further purification, unless otherwise stated. As pharmaceutically active substances, Praziquantel((*RS*)-2-(Cyclohexylcarbonyl)-2,3,4,6,7,11b-hexahydro-1*H*-pyrazino[2,1-*a*]isochinoline-4-on), Hydrochlorothiazide (6-chloro-1,1-dioxo-3,4-dihydro-2H-1,2,4-benzothiadiazine-7-sulfonamide) and Carprofen ((RS)-2-(6-Chloro-9H-carbazol-2-yl)propanoic acid) were used. Pork liver powder was used as flavouring agent. Kollidon® 30 from BASF SE (polyvinylpyrrolidone; K value of 30) was used as binding agent. Ac-Di-Sol® SD-711 Croscarmellose Sodium (cross-linked sodium carboxymethyl cellulose) was used as gel-forming agent. Pearlitol® 160C (Mannitol; particle size mean diameter 160 μm) was used as filler. Gelucire® 48/16 or 50/13 was used as surfactant.

### Manufacturing Process

In the manufacturing process of the Examples, all solid ingredients were mixed well in a drum mixer , passed twice through 0.5 mm mesh and added to the gravimetric dossier feeder (K-CL-24-KT 20, K-Tron, Niederlenz, Switzerland)

of the twin-screw extruder (Thermo Pharma HME 16, Thermo Fisher Scientific, Karlsruhe, Germany). Total length of screws was 64 cm (= 40 D). The extruder set-up and screw configuration applied in the Examples and Comparative Examples were as summarized below:

Barrel: barrel zone 1

Zone-configuration: zone 1 – powder feeding

zone 2 – preswelling liquid dosing (water)

zone 6 – swelling liquid dosing (glycerol anhydrous)

Screw configuration:

| | | | |
|---|---|---|---|
| **Engine block** | 4 D | FE (2 L/D) | |
| | 7 D | FE (1 L/D) | |
| | 2 D | KB 1 | |
| | 2 D | FE (1 L/D) | |
| | 2.5 D | KB 2 | |
| | 8 D | FE (1 L/D) | |
| | 1.5 D | KB 3 | |
| | 2 D | FE (1 L/D) | |
| | 1 D | DF (1 L/D) | |
| | 2 D | FE (1 L/D) | |
| | 1.5 D | KB 4 rev | |
| | 1 D | FE (1 L/D) | |
| | 1 D | DF (1 L/D) | |
| | 4 D | FE (1 L/D) | **Die** |
| | 0.5 D | FE (0.5 L/D) | |
| Length (total) | 40 D | | |

F = flat kneading disk

S = pointed kneading disk

DF = distributive flow element

FE = conveying elements

KB = kneading block

| | | | | | | | | | | |
|---|---|---|---|---|---|---|---|---|---|---|
| **Kneading block 1** | S(0°) | F (30°) | F (60°) | S (90°) | F (90°) | S (90°) | S (60°) | S (60°) | | |
| **Kneading block 2** | S(0°) | F (30°) | S (30°) | S (60°) | S (60°) | S (60°) | S (60°) | S (60°) | F (90°) | S (90°) |

(continued)

| | | | | | | | |
|---|---|---|---|---|---|---|---|
| **Kneading block 3** | S(0°) | F (30°) | S (30°) | S (60°) | F (90°) | S (90°) | |
| **Kneading block 4 "reverse"** | S(0°) | F (-30°) | F (-60°) | F (-60°) | F (-60°) | S (-90°) | |

| | | |
|---|---|---|
| Screw length: | 640 mm (= 40 D) | |
| Screw diameter: | 16 mm (= 1 D) | |
| Extrusion parameters: | | |
| | barrel temperature: | 40 °C (except zone 1 and die: not heated) |
| | Screw speed: | 100 rpm |
| | Powder feed: | 10 g/min |

Initially the screws were wetted by letting water into the extruder at the rate of 1.81 g/min into zone 2 until the entire screw was wet and liquid came out of the nozzle. Later, powder blend was delivered to zone 1 of the extruder at the rate of 10 g/min by the dossier. Thereafter, glycerol anhydrous was added to zone 6 of the extruder at the rate of 8 g/min and the blend was mixed homogeneously. After a lag period of few minutes (ca. 10 min), a soft homogeneous and brown wet mass was extruded through the 10 x 5 mm slit die/nozzle. The entire barrel was heated and maintained at 40°C except the zone 1 and the nozzle. Screws were rotated at 100 rpm. The output from the extruder was 19.81 g/min.

Example 1

For the preparation of a formed body, a mixture comprising the components of Table 1 was first prepared in a mixing drum and then added to a gravimetric dossier feeder (K-CL-24-KT 20, K-Tron, Niederlenz, Switzerland) of a twin-screw extruder (Thermo Pharma HME 16, ThermoFisher Scientific, Karlsruhe, Germany). The total length of the screws in the extruder were 64 cm (= 40 D).

Table 1: Components for the solid mixture; amount in wt.%, based on the total weight of the solid mixture

| **Ingredient** | **Amount [wt.%]** |
|---|---|
| Praziquantel | 6.99 |
| Pork liver powder | 20.40 |
| Ac-Di-Sol® SD-711 | 14.57 |
| Pearlitol® 160C | 48.95 |
| Kollidon® 30 | 9.09 |

The solid mixture of Table 1 was then fed into zone 1 of an extruder using the gravimetric dossier feeder. The solid mixture was fed into the extruder at a rate of 10 g/min. Water was added into zone 2 of the extruder using a pump at a rate of 1.81 g/min. In zones 2 to 5 of the extruder, water and the solid mixture were kneaded thoroughly. Then, anhydrous glycerol was added to this mixture at a rate of 8.0 g/min into zone 6 of the extruder. After a lag period of few minutes (ca. 10 min), a soft homogeneous and brown wet mass is extruded through the 10 x 5 mm (width x height) slit die/nozzle. The screws of the extruder were rotated at a speed of 100 rpm.

The temperature profile of the different zones of the extruder during the extrusion process is shown in Table 2:

Table 2: Temperature profile during the extrusion process of Example 1

| Zone | 10 | 9 | 8 | 7 | 6 | 5 | 4 | 3 | **2** | 1 |
|---|---|---|---|---|---|---|---|---|---|---|
| Temp. [°C] | 40 | 40 | 40 | 40 | 40 | 40 | 40 | 40 | 40 | 25 |

Examples 2 and 3

In Examples 2 and 3, formed bodies were prepared in the same way as in Example 1 with small changes in the composition of the solid mixture. Some of the polyvinylpyrrolidone (Kollidon® 30) was replaced with Gelucire with different HLB values. The composition of the solid mixtures for examples 2 and 3 are shown in Table 3.

Table 3: Components for the solid mixture of examples 2 and 3; amount in wt.%, based on the total weight of the solid mixture

| Ingredient | Example 2 Amount [wt.%] | Example 3 Amount [wt.%] |
|---|---|---|
| Praziquantel | 6.99 | 6.99 |
| Pork liver powder | 20.40 | 20.40 |
| Ac-Di-Sol® SD-711 | 14.57 | 14.57 |
| Pearlitol® 160C | 48.95 | 48.95 |
| Kollidon® 30 | 2.10 | 2.10 |
| Gelucire® 48/16 | 6.99 | - |
| Gelucire® 50/13 | - | 6.99 |

The temperature profile of the different zones of the extruder during the extrusion process is shown in Table 4.

Table 4: Temperature profile during the extrusion process of Examples 2 and 3

| Zone | 10 | 9 | 8 | 7 | 6 | 5 | 4 | 3 | 2 | 1 |
|---|---|---|---|---|---|---|---|---|---|---|
| Temp. [°C] | 20 | 35 | 60 | 60 | 60 | 60 | 60 | 50 | 30 | 25 |

Examples 4 and 5

In Examples 4 and 5, formed bodies were prepared in the same way as in Example 1. However, praziquantel was replaced with hydrochlorothiazide (Example 4) or carprofen (Example 5). Also, the same amount of active ingredient was used as in the case of praziquantel in Example 1.

Hardness, disintegration properties and water activity of the extrudates obtained in Examples 1 to 5 were determined in the following fashion:

*1) Determination of the hardness of the formed bodies by means of a texture analyser*

Name of device: TA.XTplus, Stable Micro Systems Ltd., UK
Software: "Exponent"-Software
Measuring device: aluminium stamp (round), diameter 0.5 cm ("Indenter")

Parameters:

Sample size: ca. 3 cm in length, width and height of the sample depend on the nozzle of the extruder

Starting position: 15 mm above sample plate
Test mode: Compression test
Test destination: 25 % deformation ("strain")
Pre-test speed: 3.0 mm/s

(continued)

| | |
|---|---|
| Test speed: | 1.0 mm/s |
| Post-test speed: | 1.0 mm/s |
| Number of cycles: | **2** test cycles |
| Trigger force: | 0.049 N (corresponds to 1 g) |

Evaluation:

| | |
|---|---|
| Number of measurements: | 3 |
| "Quick calculation" | Peak Positive Force |
| | Peak Negative Force |

Procedure:

A formed body (extrudate) was placed on the sample plate at the beginning of the test in such manner that it was in a position amidst the aluminium stamp. The parameters were set as stated above via the software, and the measurement was started.

When the measurement was started, the measuring device, which was screwed to the force cell, was moved downwards with 3 mm/s (pre-test speed). As soon as the measuring device was in contact with the sample (the trigger is a force > 0.049 N), the monitoring of the force [N] in dependency to the time [s] and the position of the aluminium stamp [mm] was started. The aluminium stamp penetrated the sample only that deep as is required to achieve a 25 % deformation in relation to the initial sample height. The penetration by the stamp took place with a speed of 1.0 mm/s (test speed). As a deformation of 25 % was achieved, the stamp returned to its initial position with a speed of 1.0 mm/s (post-test speed) until the initial height of 15 mm was reached. As soon as the stamp was in the starting position, the next cycle of the measurement started (herein, two cycles means that the measurement of hardness is performed two times, with the second measurement directly following the first measurement) using the same sample.

When the stamp penetrated the sample during the first cycle, the monitoring of the force which had to be applied for the deformation of the sample in dependency to timewas started (X-axis → time; Y-axis → force). The monitoring of force versus time was finished as soon as the aluminium stamp reaches the starting position after the second cycle.

In total, three samples were characterized as described above for each of the Examples given.

For the evaluation of the measurement results, the function "Quick calculation" of above-mentioned software was applied. Herein, the evaluation parameters "Peak Positive Force" and "Peak Negative Force" were activated and the measurements were analyzed by the software. Resulting from this, the positive and negative force of each cycle were provided separately as an average value with standard deviation as output by the software.

After each measurement, the sample plate and the aluminium stamp were cleaned with a paper tissue and, if required, with ethanol (aq., 70 %).

The hardness profile over the measurement time provides some information on the textural characteristics of the sample (see e.g. Szczesniak, A. S. (1963), Classification of textural characteristics, Journal of Food Science 28(4): 385-389). For example the profile of Example 1 in Fig. 5 shows that the sample is elastic with a slight plastic deformation (peak of the second cycle is lower than the first peak). Also, it can be concluded from the peak form that the sample is not brittle.

*2) Determination of the disintegration behavior of the samples*

| | |
|---|---|
| Name of Device: | PTZ AUTO 1EZ, Pharma Test Apparatebau AG, Germany |
| Sample holder: | six position "A type" disintegration basket (corresponding 2.9.1, Ph. Eur. 10,2, Test A - tablets and capsules of normal size) |

Parameters:

| | |
|---|---|
| Sample preparation: | The samples were cut into pieces of approx. 1.3 cm representing 25 mg API per chew (single dose). |
| Temperature setting: | 38 °C (± 1 °C) |
| Test volume: | 900 ml |
| Threshold: | 600 |
| Mode: | automated endpoint detection |

Procedure:

First, the samples were cut into pieces of 1.3 cm length. Then, the cut samples were each placed in a disintegration tube and weighted by a disintegration disc for automated endpoint detection. To start a measurement, the PTZ AUTO 1EZ was started and temperated at 38 °C by an internal heating unit. When operating temperature was reached, the measurement was started. The end of the measurement was indicated when the samples were disintegrated and each disc triggered to stop the disintegration time separately. The measured disintegration time was obtained from the device output.

*3) Determination of the water activity $a_w$ by means of the Rotronic HC2-AW-USB water activity measuring head:*

| | |
|---|---|
| Name of device: | HC2-AW-USB Measuring head, Rotronic Messgeräte GmbH, Germany |
| Software: | HW4-Software |
| Sample container: | PS-14 sample container (Rotronic) |
| Sample cup: | WP-40 (Rotronic) |
| Target parameter of measurement: | Temperature- and water activity constancy (deviation: temperature: 0.02 °C; water activity: 0.0002) |
| Sample preparation: | The samples were stored in a sealed Aluminium blister at measuring temperature for at least 12 h in order to prevent falsification of the measurement. Further, the samples were transferred to the sample containers about 1 hour before the measurement was started. |

Evaluation:

| | |
|---|---|
| Number of measurements: | 1 (continuously) |
| Method of measurement: | Direct determination of the water activity (as relative equilibrium moisture) |

Procedure:

First, each sample was directly measured after production or stored in a sealed Aluminium blister under measurement conditions for at least 12 h, as the value of water activity strongly depends on the temperature. APS-14 sample container was filled with formed bodies in such manner that the bottom is covered to the edge with formed bodies (extrudates). Then the sample cup was closed.

For starting the measurement, the HW-4 software was started. The cap covering the sample container was removed, and the lower part of the sample container was placed in the WP-40 sample cup. This was done at pace in order to prevent changes in the equilibrium of moisture above the sample, which would lead to falsification of the measurements. The HC2-AW-USB measuring head was placed on the sample cup.

The target parameters of the measurement were set via the software and the measurement was started. The measurement was continued until neither temperature nor water activity change more than the tolerance stated above. The end of the measurement was indicated by the software, and the measured water activity value was obtained from the software output.

Example 6

In Example 6 ternary croscarmellose-glycerol-water mixtures were prepared. To this aim, liquid glycerol-water pre-mixtures were prepared in different ratios and stirred with a spatula until a homogeneous suspension was generated. The ratios used in the liquid pre-mixtures of water and glycerol are summarized in Table 5.

Table 5: Used pre-mixtures of water and glycerol

| Substance | Fraction (w/w %) of liquid phase | | | | |
|---|---|---|---|---|---|
| Glycerol | 70 | 75 | 80 | 85 | 90 |
| Water | 30 | 25 | 20 | 15 | 10 |
| Glycerol:Water | 7:3 | 3:1 | 4:1 | 17:3 | 9:1 |

To obtain a gel-like structure, 1 g of croscarmellose sodium was weighted into a snap-on lid glass. 7 g of the pre-mixture of water and glycerol were directly transferred on top of the powder by a syringe. The resulting suspension was stirred with a spatula for at least 10 s to obtain a homogeneous looking mixture and subsequently analyzed for hardness of the evolving gel.

A Texture Analyser (TA.XTplus, Stable Micro Systems) equipped with 5 kg force cell and 0.5 cm aluminium punch as indentor was used. The maximum positive force applied to a sample at 25% strain was defined as hardness [N]. The hardness of each gel was recorded after a defined storage time. Initially the system was too soft for measurement. Therefore, the first 30 min of the recording were cut off.

Results

The results of the Examples are shown in figures 1 to 4.

Figure 1 shows the hardness profile for Examples 1 to 5, n = 3 ± sd. For all the Examples, the hardness of the analysed samples remained nearly unchanged over the analysed time period.

Figure 2 shows the water activity plot for Example 1 to 5, n = 1. For all the Examples, the water activity of the analysed samples remained nearly unchanged over the analysed time period.

Figure 3 shows the disintegration time displayed for Example 1 to 5, n = 3 or n = 6 ± sd. For all the Examples, disintegration times of the analysed samples were lower than 20 min were measured.

Figure 4 shows hardness profiles for gel-like croscarmellose-glycerol-water mixtures which differ from each other in their glycerol content in the liquid phase (see Table 5 of Example 6), cut-off ≤ 30 min, n =3 ± sd.

Figure 5 shows the change of hardness of a sample of Example 1 over the measurement time (two cycles).

**Claims**

**1.** Formed body for the administration to animals comprising:

a. one or more pharmaceutically active substances,
b. one or more flavouring agents,
c. one or more binding agents,
d. one or more gel-forming agents,
e. one or more fillers,
f. water, and
g. glycerol.

**2.** Formed body according to claim 1, wherein the content by weight of gel-forming agent d) in the formed body is in the range of from 0.5 to 30 wt.%, in particular from 1 to 25 wt.%, from 2 to 20 wt.%, from 3 to 17 wt.% or from 5 to 15 wt.%, based on the total weight of the formed body.

3. Formed body according to claim 1 or 2, wherein the weight ratio of gel-forming agent d) to the combined weight of water f) and glycerol g) in the formed body is in the range of from 1:2 to 1:20, in particular from 1:3 to 1:15, from 1:5 to 1:10 or from 1:6 to 1:8.

4. Formed body according to any of claims 1 to 3, wherein the weight ratio of water f) to glycerol g) in the formed body is from 5:95 to 35:65, in particular from 10:90 to 30:70 or from 15:85 to 25:75.

5. Formed body according to any of claims 1 to 4, wherein the one or more gel-forming agents d) are selected from the group of croscarmellose, low substituted carboxymethylcellulose, low substituted hydroxypropylcellulose, crospovidone and cross-linked sodium carboxymethylstarch.

6. Formed body according to any of claims 1 to 5, wherein

   a. the water content of the formed body is in the range of 0.5 to 20 wt.%, in particular 0.75 to 18 wt.%, 1.0 to 15 wt.%, 1.5 to 13 wt.% or 1.75 to 11.5 wt.%, based on the total weight of the formed body, and/or
   b. the value of water activity aw is below or equal to 0.60, in particular in the range from 0.10 to 0.60, in the range from 0.20 to 0.58, in the range from 0.30 to 0.55.

7. Formed body according to any of claims 1 to 6, wherein the one or more binding agents c) are selected from the group of polyvinylpyrrolidone, croscarmellose, hydroxypropylcellulose, hydroxypropylmethyl cellulose, hydroxyethylcellulose, pectin, pullulan, carrageenan, xanthan gum, alginate and agar agar.

8. Formed body according to any of claims 1 to 7, wherein the gel-forming agents d) comprise or consist of croscarmellose and the binding agents c) comprise or consist of polyvinylpyrrolidone.

9. Formed body according to any of claims 1 to 8, wherein at least one of the one or more pharmaceutically active substances a) is selected from the group of orally administrable systemically active veterinary pharmaceuticals.

10. Formed body according to any of claims 1 to 9, wherein the one or more flavouring agents b) are selected from the group of natural or artificial meat, poultry, fish and sea food flavours, preferably dried meat, poultry and fish powders, most preferably pig liver powder.

11. Formed body according to any of claims 1 to 10, wherein the one or more fillers e) are selected from the group of lactose, cellulose, poorly soluble inorganic salts, in particular dicalcium phosphate, solid sugar alcohols, in particular mannitol, xylitol or sorbitol and inorganic calcium, magnesium, sodium or potassium salts.

12. Formed body according to any of claims 1 to 11, containing
0.1 to 25 wt.% of one or more pharmaceutically active substances a),
1 to 25 wt.% of one or more flavouring agents b),
1 to 20 wt.% of one or more binding agents c),
0.5 to 30 wt.% of one or more gel-forming agents d),
0 to 50 wt.% of one or more fillers e),
2 to 30 wt.% water f),
5 to 65 wt.% glycerol g),
0 to 20 wt.% of one or more auxiliary additives, as for instance formulation adjuvants, lubricants, disintegrants, surfactants, humectants and preservatives, wherein the percentages add up to the sum of 100 wt.%.

13. Formed body according to any of claims 1 to 12 for use in the control of parasites in non-human animals, in particular dogs and/or cats.

14. Process for the production of aformed body according to claims 1 to 12, comprising the following steps:

   a) providing at least the following components:

      a. one or more pharmaceutically active substances,
      b. one or more flavouring agents,
      c. one or more binding agents,
      d. one or more gel-forming agents,

e. one or more fillers,
f. optionally one or more auxiliary additives, as for instance formulation adjuvants, lubricants, disintegrants, surfactants, humectants and preservatives,

b) preparing a swollen mixture by mixing the components of step a), glycerol and water,
c) forming a formed body by extrusion of the swollen mixture prepared in step b).

**15.** Process according to claim 14, wherein the step(s) b) and/or c) is/are performed in an extruder.

Fig. 1

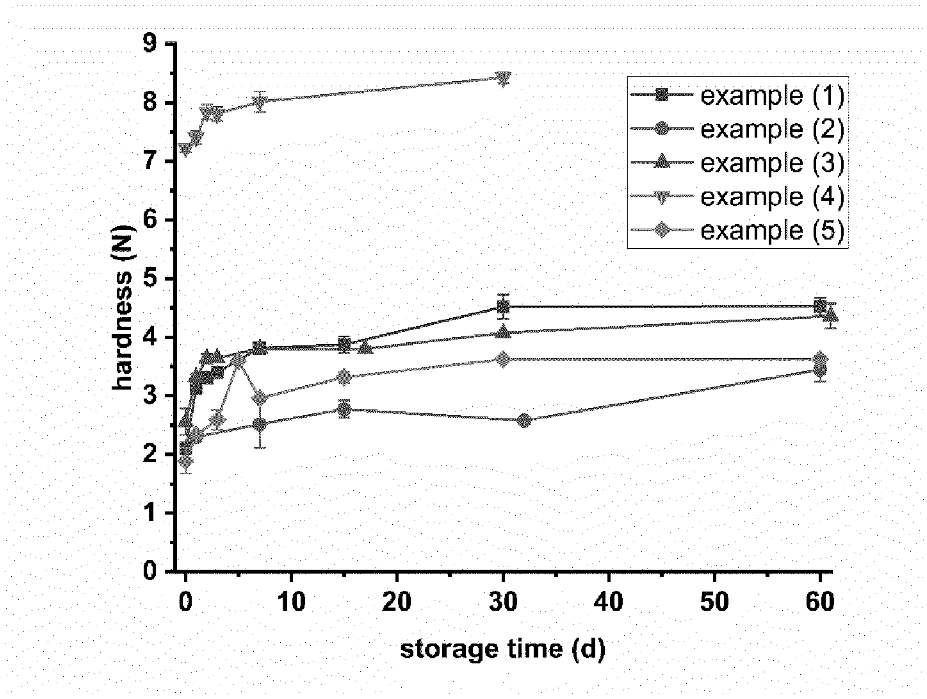

hardness (N)
storage time (d)
example (1)
example (2)
example (3)
example (4)
example (5)
0
1
2
3
4
5
6
7
8
9
0
10
20
30
40
50
60

Fig. 2

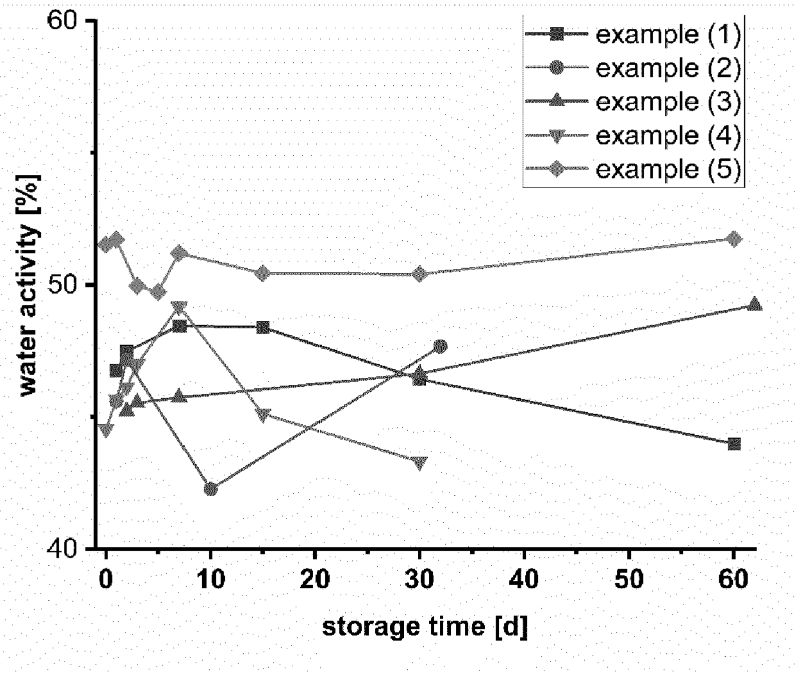
example (1)
example (2)
example (3)
example (4)
example (5)
water activity [%]
storage time [d]
60
50
40
0
10
20
30
40
50
60

Fig. 3

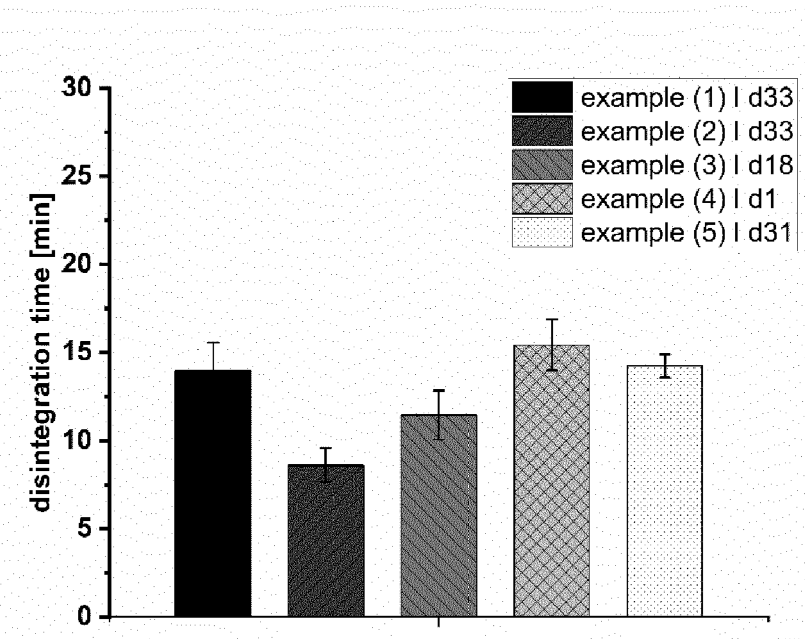
example (1) I d33
example (2) I d33
example (3) I d18
example (4) I d1
example (5) I d31
disintegration time [min]
30
25
20
15
10
5
0

Fig. 4

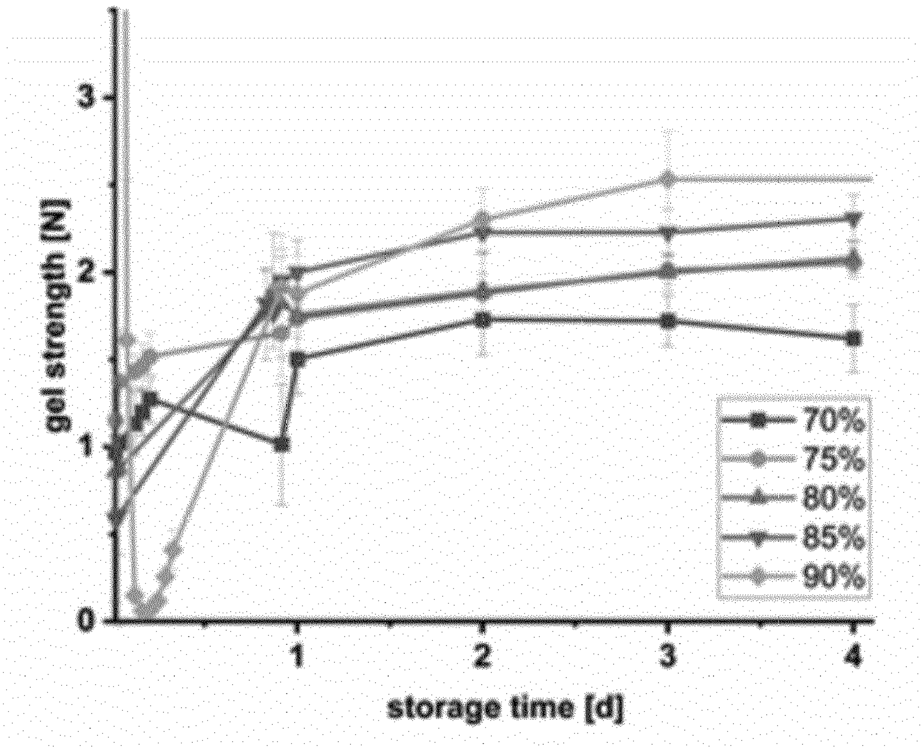
gel strength [N]
storage time [d]
70%
75%
80%
85%
90%
0
1
2
3
4

Fig. 5

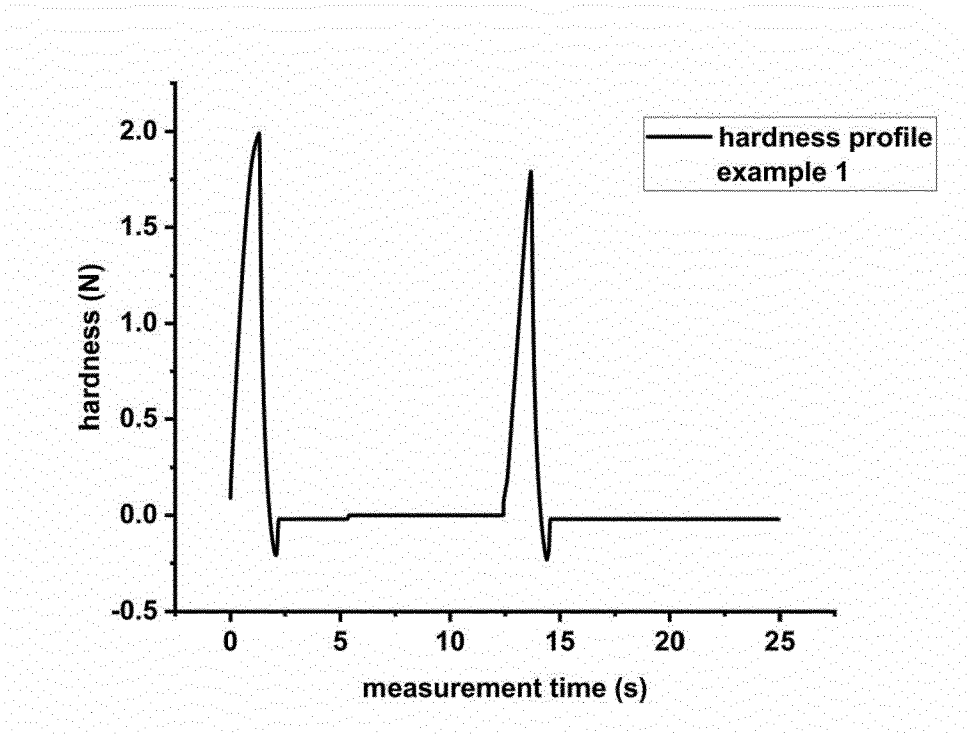
hardness profile
example 1
hardness (N)
measurement time (s)
2.0
1.5
1.0
0.5
0.0
-0.5
0
5
10
15
20
25

Europäisches Patentamt
European Patent Office
Office européen des brevets

# EUROPEAN SEARCH REPORT

Application Number
EP 20 16 1829

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/073347 A1 (ZOETIS SERVICES LLC [US]) 12 May 2016 (2016-05-12)<br>* claims 1, 2, 5, 9, 14 15 *<br>* example 12 *<br>* page 2, line 15 - page 6, line 7 *<br>* page 19, line 14 - page 20, line 13 *<br>* page 16, line 18 - page 17, line 15 *<br>* page 17, line 16 - page 17, line 2 *<br>* page 20, lines 13-19 *<br>* page 7, lines 11-12 *<br>* page 1, line 21 - page 2, line 2 *<br>----- | 1-15 | INV.<br>A61K9/16<br>A61K31/435<br>A61P33/10 |

TECHNICAL FIELDS SEARCHED (IPC)
A61K
A61P

The present search report has been drawn up for all claims

1

| Place of search | Date of completion of the search | Examiner |
|---|---|---|
| Munich | 12 August 2020 | Vázquez Lantes, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 16 1829

This annex lists the patent family members relating to the patent documents cited in the above-mentioned European search report. The members are as contained in the European Patent Office EDP file on
The European Patent Office is in no way liable for these particulars which are merely given for the purpose of information.

12-08-2020

| Patent document cited in search report | Publication date | Patent family member(s) | Publication date |
|---|---|---|---|
| WO 2016073347 A1 | 12-05-2016 | AU 2015343387 A1 | 18-05-2017 |
| | | CA 2965524 A1 | 12-05-2016 |
| | | CN 106999421 A | 01-08-2017 |
| | | EP 3215120 A1 | 13-09-2017 |
| | | JP 2017533959 A | 16-11-2017 |
| | | US 2017354593 A1 | 14-12-2017 |
| | | WO 2016073347 A1 | 12-05-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7348027 B, Rose
- WO 2004016252 A, Cleverly
- DE 69937780, Damon
- AU 2008201605 B2, Isele
- EP 1296655 B1, Kalbe
- US 6444218 B, Han, Y.D. and Park, J.B.
- US 6440450 B, Han, Y.D. and Park, J.B.
- WO 2004014143 A1, Huron, S.
- US 7955632 B2, Paulsen
- WO 2013068371 A1, Carrillo, B. and Freehauf, K.
- US 20100291245 A, Gao
- WO 2012049156 A1, Hamann, H.J. and Kanikanti, V.-R.
- US 20080075759 A1
- WO 2014141223 A1
- WO 2016073347 A1
- WO 2017194415 A1
- US 391112 A


### Non-patent literature cited in the description


- **THOMBRE, A.G.** *Advanced Drug Delivery Reviews,* 2004, vol. 56 (10), 1399-1413
- **AHMED, I. ; KASRAIAN, K.** *Advanced Drug Delivery Reviews,* 2002, vol. 54 (6), 871-882
- **HOUPT, K.A. ; SMITH, S.L.** *Canadian Veterinary Journal,* 1981, vol. 22 (4), 77-85
- **KEETELS, C.J.A.M. et al.** *Food Hydrocolloids,* 1996, vol. 10 (3), 343-353
- **FARHAT et al.** *Starch/Stärke,* 2001, vol. 53 (9), 431-436
- **VANDEPUTTE et al.** *Journal of Cereal Science,* 2003, vol. 38 (19), 61-68
- **SZCZESNIAK, A. S.** Classification of textural characteristics. *Journal of Food Science,* 1963, vol. 28 (4), 385-389
- Kollidon, Polyvinylpyrrolidone for the pharmaceutical industry. **V. BÜHLER.** BASF Pharma Ingredients. 2008